# EUROPEAN PATENT SPECIFICATION

(11) **EP 1 988 929 B1**
(45) Date of publication and mention of the grant of the patent: **21.12.2011**
(21) Application number: 07700540.3
(22) Date of filing: 31.01.2007
(51) Int. Cl.: A61L 2/07, B01J 3/04

(54) **STEAM DESK STERILIZER AND METHOD FOR CONTROLLING THE WORKING PRESSURE THEREOF**
DAMPFTISCH-STERILISATOR UND VERFAHREN ZUR STEUERUNG VON DESSEN BETRIEBSDRUCK
STERILISATEUR A VAPEUR DE TABLE ET PROCEDE DE REGULATION DE LA PRESSION DE SERVICE DE CELUI-CI

(30) Priority: 28.02.2006 IT CR20060005
(43) Date of publication of application: 12.11.2008
(73) Proprietor: Cortellini, Marinella, 26038 Torre de'Picenardi (IT)
(72) Inventor: Cortellini, Marinella, 26038 Torre de'Picenardi (IT)
(74) Representative: Marcio', Paola
(86) International application number: PCT/IB2007/000297
(87) International publication number: WO 2007/099418

(56) References cited:
- EP-A2- 0 492 056
- WO-A-98/02193
- US-A- 3 481 692
- US-A- 4 263 258
- US-B1- 6 379 613

## Description

The present invention relates to a steam desk sterilizer or "autoclave". The invention further relates to a method for controlling the working pressure, that is the steam pressure inside the sterilizing chamber, in a steam desk sterilizer.

Steam desk sterilizers are know mainly to sterilize medical and surgical instruments, especially in the field of dentistry. They are also used by other people like tattoo-artists, beauticians, etc.

Sterilization is made with saturated steam at a relatively high pressure, about 2 bar, and temperature of about 140-150 °C. Steam sterilization is generally preferred because it is effective, quick, with no risk of damaging the instruments and leaves no toxic or dangerous residual.

Desk autoclaves essentially comprise a sterilization chamber connected to an electric steam generator. In some cases, a vacuum pump is provided to extract air from the chamber. According to known technique, the sterilization cycle is controlled through a temperature probe (thermo-resistance) associated to the sterilization chamber and disposed to detect the steam temperature inside the chamber.

The following problem arises: according to applicable norms (e.g. the EU directive 97/23/CE) the project of a pressure vessel must take into account some factors, including type of equipment, fluid, normal pressure and volume. A relevant parameter is the product P·V, wherein P is the working pressure (bar) and V is the volume (liters) of the vessel. According to this parameter, it may be required a simple quality assurance by the manufacturer, or an audit by an external authority, for example to check the weldings.

More in detail, due to the sterilization chamber being directly connected to the steam generator, and the temperature probe being associated to the chamber, the chamber and the steam generator, as a whole, are considered a fired pressurized equipment falling into category II of Directive 97/23/CE. This category comprises various equipments for providing steam or superheated water above 110 °C, with volume over 2 litres, including pressure cookers.

In this case, the aforementioned directive prescribes a compulsory check by an external authority when product P·V is greater than 50.

As above, the sterilization process needs steam at about 2 bars, so that the majority of desk sterilizers are designed with a maximum capacity of 24 liters, in order to keep the product P·V below the threshold value of 50. The reason, clearly, is that an external audit is a supplementary and relevant cost for the manufacturer. US 6 379 613 relates to an autoclave wherein the steam flow from the steam generator and the sterilizing chamber is controlled by a three way valve. EP 0 492 056 discloses an autoclave in which the steam is formed directly within the sterilizing chamber.

The purpose of the invention is to solve this problem. In particular, the purpose of the invention is to realize a desk sterilizer wherein the sterilizing chamber is not subject to above-cited requirements for fired equipments.

The purposes are achieved by the desk sterilizer autoclave, and the method of claims 1 and 5 respectively. Further preferred embodiments of the invention are characterised in the remaining claims.

In a preferred embodiment, said steam generator is a cylindrical body having a volume less than 2 liters. Said probe is, for example, a PT100 thermo-resistance sensitive to the temperature of the steam inside the sterilizing chamber.

The autoclave comprises an electronic control system on a suitable electronic card. Said control system is provided with a memory device for storing the theoretical values of pressure and temperature of the saturated steam (saturation curve). This way, the pressure can be detected and controlled in an indirect way through the temperature signal of the aforementioned probe.

A further aspect of the invention, then, is a method for indirect control of the working pressure in a desk sterilizer comprising a sterilizing chamber connected to a steam generator, wherein the steam pressure inside said sterilizing chamber is controlled through a temperature signal provided by a probe directly associated to the steam generator.

Said method implies a correlation of pressure to temperature through the temperature-pressure values of the saturated steam, stored in a suitable memory.

The advantage of the invention is that the sterilizing chamber is simply considered as a pressure vessel under cat. I of Dir. 97/23/CE, and is not subject to above requirements. Autoclaves with working pressure of 2 bars and sterilizing chamber over the current standard of 24 liters can be made without the expensive need of a check by an external authority.

Making the steam generator as a cylindrical body of less than two liters, it is not subject to norm requirements further than due care by the manufacturer.

Further aspects of the invention will become apparent with the help of the following description and figures, wherein:

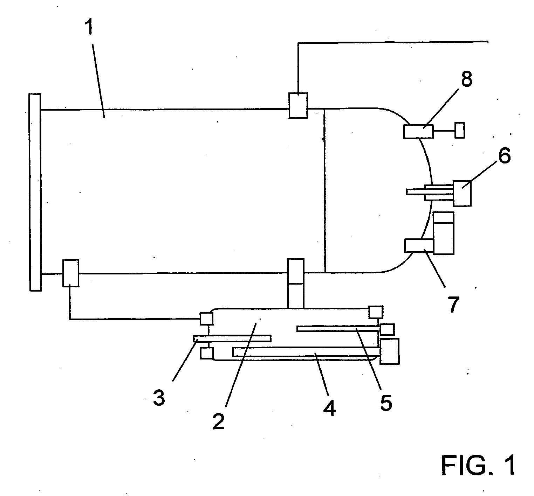

Fig. 1 is a functional diagram of the sterilizing chamber and steam generator of an autoclave according to the invention;
Fig. 2 is a general diagram of the same autoclave, showing besides the sterilizing chamber and steam generator, a number of other components and accessories;
Fig. 3 is an electric diagram of the autoclave, according to a preferred embodiment.

Referring to figures, the invention relates to a desk sterilizing autoclave comprising a sterilizing chamber 1 and a steam generator 2 directly connected to it.

The steam generator 2 is preferably a cylindrical body having a volume less than two liters.

A safety thermostat 3, a cartridge resistor 4 and a thermo-resistance 5 are associated to steam generator 2. Said cartridge resistor 4 is charged of steam generation and for common equipments is rated to 1800 W about; the safety thermostat 3 may be set e.g. to 200 °C.

Thermo-resistance 5 is sensitive to the temperature of the steam inside the steam generator 2. The signal from said thermo-resistance 5 is used for indirect control of the steam pressure inside the sterilizing chamber 1, as will be detailed below.

A pressure sensor 6, a safety valve 7 and a further thermo-resistance 8 are associated, on the contrary, directly to the sterilizing chamber 1.

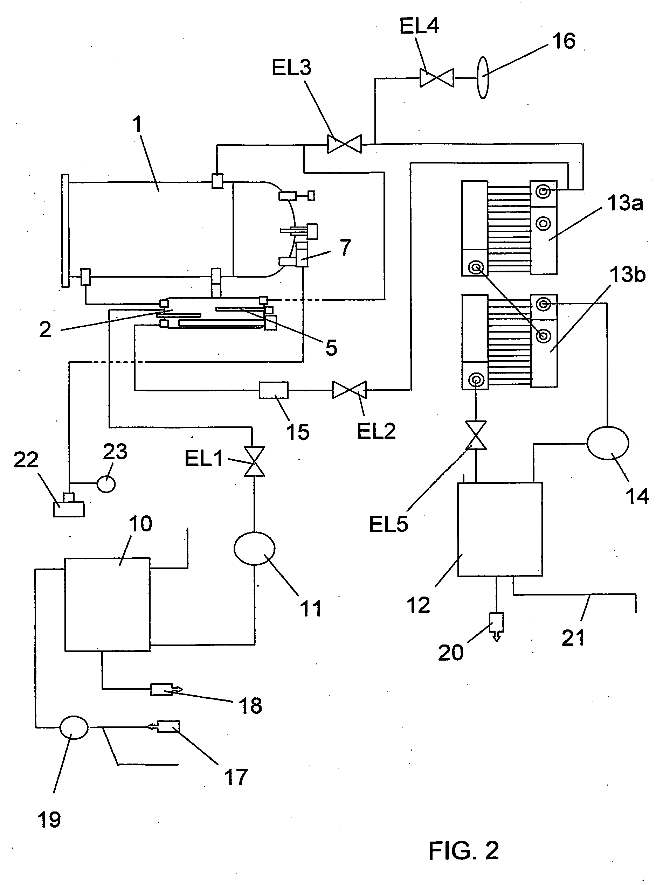

Referring now to Fig. 2, further components of the autoclave are the following: a reservoir 10 containing the water for steam generation; a pump 11 to feed the generator 2; a discharge reservoir 12; two radiators 13a and 13b connected in series; a vacuum pump 14 to evacuate air from sterilizing chamber 1, a filter 15 to stop impurities, a bacteriologic filter 16.

The above-cited components are connected by tubes fitted with suitable solenoid valves, namely: a solenoid valve EL1 normally close between reservoir 10 and steam generator 2; a solenoid valve EL2, normally open, between steam generator 2 and radiators 13a, 13b; a solenoid valve EL3 normally close between chamber 1 and radiators 13a, 13b; a solenoid valve EL4 downstream the bacteriologic filter 16; a solenoid valve EL5, normally open, between the radiators 13a, 13b and the discharge reservoir 12.

The reservoir 10 is fitted with a feeding faucet 17, a discharge faucet 18 and a feeding pump 19 preferably connectable to a deionizer for automatically feed de-ionized water.

The vacuum pump 14 and the bacteriologic filter 16 allow forced ventilation and complete drying of the instruments. The vacuum pump, moreover, allow to evacuate almost totally the air and penetration of sterilizing steam in materials with some porosity and/or in the most critical instruments like turbines for dentists.

The discharge reservoir 12 is equipped with a discharge faucet 20 and preferably is also equipped with an automatic discharge 21.

The safety valve 7 is connected to a manometer 22 with pressure transducer 23.

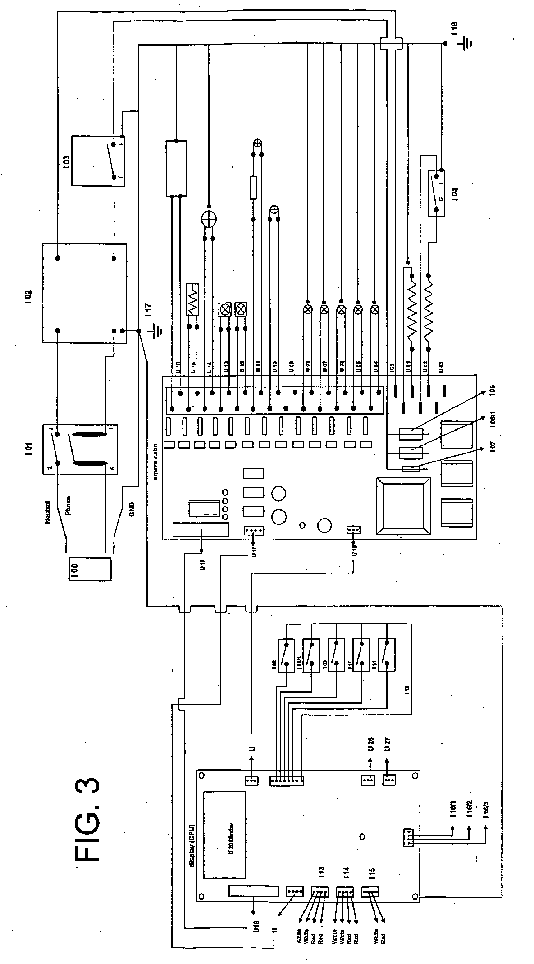

Fig. 3 is the electrical scheme of the appareil. The following table is the reference list of Fig. 3 as regards the outputs (U).

| N° | Item | Specifications |
|---|---|---|
| U 01 | Steam-generation resistance | 230 V (AC) 1800 W 50Hz |
| U 02 | Band Resistance | 230 V (AC) 1000 W 50Hz |
| U 03 | Available | |
| U 04 | Valve 1 N.C. | 230 V (AC) 50 Hz 20 W |
| U 05 | Valve 2 N.O. | 230 V (AC) 50 Hz 15 W |
| U 06 | Valve 3 N.C. | 230 V (AC) 50 Hz 20 W |
| U 07 | Valve 4 N.C. | 230 V (AC) 50 Hz 20 W |
| U 08 | Valve 5 N.O. | 230 V (AC) 50 Hz 15 W |
| U 09 | Available | |
| U 10 | Pump steam generator | 230 V (AC) 50 Hz 48 W |
| U 11 | Pump reservoir | 230 V (AC) 50 Hz 48 W |
| U 12 | Fan radiator 1 | 220-240 V (AC) 50/60Hz 32,2 W |
| U 13 | Fan radiator 2 | 220-240 V (AC) 50/60Hz 32,2 W |
| U 14 | Vacuum pump | 230 V (AC) 50 Hz 95W |
| U 15 | Thermo actuator | 110/240 V (AC) 50/60 HZ 27,6 W |
| U 16 | Printer power supply | 230 V (AC) - 5 V. (DC) 50 Hz 25 W |
| U 17 | Display power supply | 0-15 V (4 pins) |
| U 18 | Display power supply | 0-15 V (3 pins) |
| U 19 | Flat cable | 20 wires |
| U 20 | Display | LCD 8x16 |
| U 26 | Serial port - printer | RS 232 |
| U 27 | Serial port - PC | RS 232 |

The following table relates to inputs (I).

| N° | Item | Specifications |
|---|---|---|
| I 00 | Unipolar supply cable | 450/750 V 3x2,5 mm2 |
| | Schuko | 16 A 250 V |
| I 01 | Bipolar switch green light | 16A 250V |
| I 02 | Filter | 10DRCG5 10A double cell |
| I 03 | Safety thermostat | Set to 200°C 16A 250V. |
| | steam generator resistance | Manual reset |
| I 04 | Safety thermostat band | Set to 140°C 16A 250V. |
| | resistance | Manual reset |
| I 05 | Power supply card | 220 V. K 10% (AC) 50 Hz |
| I 06 | N. 1 Fuse 10x38 (ceramic) | 10A 500V |
| I 06/1 | N. 1 Fuse 10x38 (ceramic) | 4A 500V |
| I 07 | N. 1 Fuse 5x20 (ceramic + quartz) | 1,6A 250V |
| I 08 | Sensor 2 levels (max) reservoir | N.O. (MAX) |
| I 08/1 | Sensor 2 levels (min) reservoir | N.C. (MIN.) |
| I 09 | Sensor 1 level (max) discharge reservoir | N.O. (MAX) |
| I 10 | Door microswitch | N.O.15 A 250 V |
| I 11 | Safety microswitch | N.O.15 A 250 V |
| I 12 | Common sensors and microswitches | Common |
| I 13 | Steam generator temperature sensor | PT 100 4 wires |
| I 14 | Chamber temperature sensor | PT 100 4 wires |
| I 15 | Band resistance temp. sensor | PT 100 2 wires |
| I 16/1 | " - " pressure sensor | Scale -1 /+ 3 bar |
| I 16/2 | " + " pressure sensor | 0-10 V |
| I 16/3 | Pressure sensor signal | |
| I 17 | Earth | Inox screw 4 MA |
| I 18 | Earth | Inox screw 4 MA |

The autoclave also comprises an electronic control system on a suitable card. Said card comprises a memory which contains the temperature-pressure values of saturated water steam; user-selectable programs or cycles for sterilization are also stored. The steam generator is operated through a PID system.

The operation, in brief, is as follows: after the filling with water of the steam generator 2 by the pump 11, the control system turns on the resistance 4 of steam generator 2. This is allowed only if temperature of generator 2, read by thermo-resistance 5, is less than 150 °C, the temperature of the sterilizing chamber, read by thermo-resistance 8, is less than 140 °C and pressure inside said chamber, read by pressure sensor 6, is less than 2.5 bar. In case of dry operation, the generator is protected by thermostat 3 set to 200 °C.

When the temperature and pressure for sterilization are reached, according to the user-selected cycle, the control system keeps the correct temperature and pressure, to follow the selected cycle.

The temperature of the chamber and the theoretical steam temperature, which comes from a comparison between the measured temperature and pressure and data stored in the memory of the card, must satisfy the following conditions: the temperature must not be less than that of sterilization; difference between the measured and the theoretical temperature must not exceed 2 °C (saturation check).

Small dimensions of steam generator 2 allow for a very quick response: in practice, the amount of steam which is needed to avoid that temperature fall below the minimum value required by sterilization program can be rapidly produced by the generator.

The resistances are operated by TRIAC which, unlike common relais, in case of malfunction do not continue to supply power. Thus, there is no risk of uncontrolled power supply.

The autoclave comprises a number of alarms, to provide a safe operation. The following is a list of some examples of alarms, citing possible conditions of intervention.

A1: failure of temperature sensor of the chamber, always on, temperature of the chamber < 0 °C or >180 °C.

A2: failure of temperature sensor of steam generator, always on, temperature of steam < 0 °C or >180 °C.

A3: failure of resistance temperature sensor, always on, temperature < 0 °C or >180 °C.

A4: failure of pressure transducer, always on, pressure < -1 bar or > 3 bar.

A5: door open, active after cycle start and during cycle, door microswitch inactive.

A6: safety not active, 3 mins. after start and during cycle, safety microswitch not active.

A7: manual interruption, active during cycles 3 seconds after pressing the RESET and START buttons.

A8: timeout alarm, active during cycles, duration timeout of the phase according to predetermined tables.

A9: timeout of reservoir charge, active during the automatic charge procedure; max level signal not active after 10 mins.

A10: black-out automatic switch-on, active during all cycles, power failure during cycles, with following three attempts and switch on without intervention of alarm or warnings.

A11: black-out, active during all cycles, after three attempts of automatic power on without intervention of alarm or warnings. Visualization must take place when power is restored.

A12: excessive humidity during first 300 seconds, active during the "vacuum test" cycle.

A13: air infiltration last 600 seconds, active during the "vacuum test" cycle, for pressure raise more than 0,13 kPa/min.

A14: alarm of low temperature of the chamber during sterilization. A15: alarm of high temperature of the chamber.

A16: alarm of insufficient saturation, active during the sterilization time, when theoretical temperature (obtained through the theroetical saturation curve of the water) and temperature read in the chamber differ of more than 2 °C.

A17: low steam temperature with respect to the preset cycle.

A18: high steam temperature with respect to the preset cycle.

A19: high pressure in the chamber, the alarm is active during all cycles and when pressure exceeds 350 kPa.

For the temperature sensors, alarms A1, A2 and A3 are always active.

During a cycle, if the pressure in the chamber and steam generator exceed the preset value, the safety valve intervenes at 2.4 bar.

Should the valve not open, for further safety, there is provided the alarm A19 which interrupts the running cycle, cutting the power to all outputs, when pressure in the chamber and generator reaches 2,5 bar.

In case of failure of alarm A19, with resistance of the generator still powered, the PID cuts out the power when temperature of the generator is over 150 °C, or temperature of the chamber > 140 °C (that is max pressure of 2.6 bar according to Mollier diagram) and pressure of the chamber > 2.5 bar.

Alarms A14 and A15 keep the temperature of the chamber under control, as explained above.

The A16 alarm compares the temperature of the chamber and theoretical temperature. At high pressures, the theoretical temperature would deviate of more than 2 degrees as prescribed, and as a consequence this alarm would intervene interrupting the cycle and cutting the power of all devices.

The alarms A17 and A18 check the theoretical steam temperature, comparing temperature and pressure with the Mollier table stored in the card. Then, in case of uncorrect high pressure in the chamber the intervention of these two alarms guarantees that the cycle is interrupted.

For failed intervention of above cited alarms, when pressure is less than -1 or more than 3 bar, alarm A4 "failure of pressure transducer" intervenes, interrupting the current cycle.

Hence, the autoclave according to the invention meets the above purposes, operating with maximum safety.

## Claims

1. A desk sterilizer autoclave, comprising at least a sterilizing chamber (1), a steam generator (2) connected to said chamber (1) and at least one temperature probe, consisting of a thermo-resistance (5) for controlling the steam generation and being directly associated to said steam generator (2) and is sensitive to the steam temperature within said steam generator, said autoclave comprising an electronic control system on an electronic card having a memory device containing theoretical values of pressure and temperature of saturated steam, defined by the saturation curve, wherein the temperature signal provided by the thermo-resistance (5) is correlated to the pressure value of the saturated steam curve, being thus used for the indirect control of the steam pressure inside said sterilizing chamber (1).

2. Autoclave according to claim 1, **characterized in that** said steam generator is made of a cylindrical body having internal volume less than two liters.

3. Autoclave according to claim 1 or 2, **characterized In that** a pressure sensor (6), a safety valve (7) and a further temperature probe (8) are associated to said sterilizing chamber (1).

4. Autoclave according to any one of preceding claims, **characterized by** comprising a reservoir (10) containing water to produce steam; a generator-feeding pump (11); a discharge water reservoir (12); two radiators (13a, 13b) connected In series; a vacuum pump (14) for evacuating air from said sterilization chamber (1).

5. Method for indirect control of the working pressure in a desk sterilizer autoclave comprising a sterilization chamber (1) connected to a steam generator (2) and an electronic control system on an electronic card having a memory device containing theoretical values of pressure and temperature of saturated steam, defined by the saturation curve, wherein the steam pressure inside said sterilization chamber (1) is controlled by means of a temperature signal provided by a thermo-resistance (5) directly associated to said steam generator (2) and sensitive to the steam temperature within said steam generator, and the steam pressure in the sterilization chamber (1) is correlated to the temperature by means of the pressure-temperature values of the saturated steam, stored in said memory device.

## Patentansprüche

1. Ein Tisch-Sterilisator-Autoklav, umfassend mindestens eine Sterilisierkammer (1), einen Dampferzeuger (2), der mit besagter Kammer (1) verbunden ist und mindestens einen Temperaturfühler, bestehend aus einem thermischen Widerstand (5) zur Dampfkontrolle und, da er an den besagten Dampferzeuger (2) direkt angeschlossen ist und auf die Dampftemperatur in besagtem Dampferzeuger empfindlich reagiert, umfasst besagter Autoklav ein elektronisches Kontrollsystem auf einer elektronischen Karte mit einem Datenspeicher, der theoretische Druckwerte und die durch die Sättigungskurve definierte Temperatur des gesättigten Dampfes enthält, wobei das Temperatursignal, das vom thermischen Widerstand (5) geliefert wird, in Beziehung zum Druckwert der gesättigten Dampfkurve steht und auf diese Weise zur indirekten Kontrolle des Dampfdrucks innerhalb der besagten Sterilisierkammer (1) benutzt wird.

2. Autoklav nach Anspruch 1, **gekennzeichnet dadurch, dass** besagter Dampferzeuger aus einem zylinderförmigen Körper besteht, dessen Innenvolumen weniger als zwei Liter beträgt.

3. Autoklav nach Anspruch 1 oder 2, **gekennzeichnet dadurch, dass** ein Drucksensor (6), ein Sicherheitsventil (7) und ein weiterer Temperaturfühler (8) mit besagter Sterilisierkammer (1) in Verbindung gebracht werden.

4. Autoklav nach einem jeglichen der vorherigen Ansprüche, **gekennzeichnet dadurch, dass** er einen Wasserbehälter (10) zur Dampferzeugung; eine Pumpe (11) zum Füllen des Dampferzeugers; einen Wasserbehälterabfluss (12); zwei in Serie angeschlossene Radiatoren (13a, 13b); eine Vakuumpumpe (14) zum Ausstoßen der Luft aus der besagten Sterilisierkammer (1) umfasst.

5. Methode zur indirekten Kontrolle des Arbeitsdrucks in einem Tisch-Sterilisator-Autoklav, der eine Sterilisierkammer (1) umfasst, und verbunden ist mit einem Dampferzeuger (2) und einem elektronischen Kontrollsystem auf einer elektronischen Karte mit einem Datenspeicher, der theoretische Druckwerte und die durch die Sättigungskurve definierte Temperatur des gesättigten Dampfes enthält, wobei der Dampfdruck in der besagten Sterilisierkammer (1) mittels eines von einem thermischen Widerstand (5) gelieferten Temperatursignals kontrolliert wird, das direkt in Verbindung steht mit besagtem Dampferzeuger (2) und in besagtem Dampferzeuger auf die Dampftemperatur empfindlich reagiert, und der Dampfdruck in der Sterilisierkammer (1) zur Temperatur in Beziehung steht mittels der Druck-Temperatur-Werte des gesättigten Dampfes, die in besagtem Datenspeicher gespeichert sind.

## Revendications

1. Un autoclave stérilisateur de bureau, comprenant au moins une chambre de stérilisation (1), un générateur de vapeur (2) relié à la dite chambre (1) et au moins une sonde de température, consistant en une résistance thermique (5) pour contrôler la production de vapeur et étant directement associé au dit générateur de vapeur (2) et est sensible à la température de la vapeur dans le dit générateur de vapeur, le dit autoclave comprenant un système de contrôle électronique sur une carte électronique ayant un dispositif de mémoire contenant des valeurs théoriques de pression et température de vapeur saturée, définies par la courbe de saturation, où le signal de température procuré par la résistance thermique (5) est corrélée à la valeur de pression de la courbe de vapeur saturée, ainsi utilisée pour le contrôle indirect de la pression de la vapeur dans la dite chambre de stérilisation (1).

2. Autoclave selon la revendication 1, **caractérisé par le fait que** le dit générateur de vapeur est composé d'un corps cylindrique ayant un volume interne de moins de deux litres.

3. Autoclave selon la revendication 1 ou 2, **caractérisé par le fait que** un capteur de pression (6), une soupape de sécurité (7) et un détecteur de température supplémentaire (8) sont associés à la dite chambre de stérilisation (1).

4. Autoclave selon n'importe quelle des revendications qui précèdent, **caractérisé par le fait qu'**il comprend un réservoir (10) contenant de l'eau pour produire de la vapeur ; un générateur/pompe d'alimentation (11) ; un réservoir d'eau de décharge (12) ; deux radiateurs (13a, 13b) reliés en série ; une pompe à vide (14) pour évacuer l'air de la dite chambre de stérilisation (1).

5. Méthode de contrôle indirect de la pression de fonctionnement dans un autoclave de stérilisation de bureau comprenant une chambre de stérilisation (1) connectée à un générateur de vapeur (2) et un système de contrôle électronique sur une carte électronique ayant un dispositif de mémoire contenant des valeurs théoriques de pression et de température de la vapeur saturée, définies par la courbe de saturation, où la pression de la vapeur dans la dite chambre de stérilisation (1) est contrôlée au moyen d'un signal de température procuré par une résistance thermique (5) directement associée au dit générateur de vapeur (2) et sensible à la température de la vapeur dans le dit générateur de vapeur, et la pression de la vapeur dans la chambre de stérilisation (1) est corrélée à la température au moyen des valeurs de pression/température de la vapeur saturée, enregistrées dans le dit dispositif de mémoire.
